# EUROPEAN PATENT APPLICATION

(11) **EP 3 103 379 A1**
(43) Date of publication of application: **14.12.2016**
(21) Application number: 15790001.0
(22) Date of filing: 07.05.2015
(51) Int. Cl.: A61B 1/04, G02B 23/24

(54) **ELECTRONIC ENDOSCOPE**

(30) Priority: 08.05.2014 JP 2014097061
(71) Applicant: OLYMPUS CORPORATION, Hachioji-shi Tokyo 192-8507 (JP)
(72) Inventor: TANABE Takahiro, Tokyo 192-8507 (JP); SAKUMA Koji, Tokyo 192-8507 (JP)
(74) Representative: Gunzelmann, Rainer
(86) International application number: PCT/JP2015/063191
(87) International publication number: WO 2015/170701

(57) **Abstract**

An electronic endoscope 2 transmits an image pickup signal obtained by an image pickup section 14 and converted into digital serial data, as a differential signal, to a processor 3, and includes a transmission section 15 for transmitting the differential signal to the processor 3, and a filter section 16 arranged at an output stage of the transmission section 15, wherein a constant of the filter section is adjusted according to a loss in a transmission channel including a cable such that a quality of output waveform to the processor 3 is fixed.

## Description

### Technical Field

The present invention relates to an electronic endoscope, and more particularly to an electronic endoscope that transmits an image pickup signal obtained by an image pickup section and converted into digital serial data as a differential signal to a processor.

### Background Art

Endoscope systems that include an electronic endoscope for picking up an image of an object inside a subject to be examined, a processor for generating an observation image of the object whose image has been picked up with the electronic endoscope, and the like have been widely used in medical fields and industrial fields.

The electronic endoscope is required to serialize A/D converted image pickup data and transmit the serialized data over a distance of several meters to a processor which includes a reception circuit. In recent years, ISI (Intersymbol interference), which is waveform distortion caused by interference between adjacent symbols, increasingly occurs due to a need for an increase in the number of pixels and reduction in diameter, which leads to a difficulty in securing transmission quality. In conventional systems, a buffer with an equalizer is provided at a connector portion located at a position closer to a processor. However, it is desirable to intensively arrange the electronic circuits in order to reduce the cost.

As a method of intensively arranging the electronic circuits, it can be considered to arrange an equalizer in the processor having a reception circuit. However, in order to gear the equalizer to electronic endoscopes having insertion portions of various lengths, it is necessary to provide a plurality of equalizers corresponding to the respective lengths, which results in an increase of the circuit scale in the processor. In addition, the output waveform from the electronic endoscope is in the state before the correction of intersymbol interference, which results in a difficulty in quality control.

Therefore, Japanese Patent Application Laid-Open Publication No. 2011-35630, for example, discloses a camera head separated type camera device configured such that the camera head and camera control unit are separated from each other. The camera head separated type camera device disclosed in the Japanese Patent Application Laid-Open Publication No. 2011-35630 includes: a voltage measurement portion that measures voltage drop due to a cable; a table that shows a relation between the voltage drop and each correction value that are stored in advance; a correction control portion that extracts a correction value from the table according to the measured voltage drop; an LVDS control portion that performs adjustment of the LVDS signal waveform based on the correction value; and an LVDS conversion driver that generates a waveform of the LVDS signal to transmit the generated waveform to the camera control unit, wherein an accurate video signal is reproduced in the camera control unit.

According to the camera head separated type camera device disclosed in the Japanese Patent Application Laid-Open Publication No. 2011-35630, the transmission-side camera head has a pre-emphasis function, to thereby enable the intersymbol interference to be suppressed. However, a power source is required for adjustment of the voltage amplitude of the signal waveform and reinforcement of the rising (falling) portion of the signal waveform, which leads to a problem of increase in power consumption.

As a measure for low power consumption, for example, some electronic endoscopes include a de-emphasis function in the transmission-side camera head. However, such a de-emphasis function is assumed to be used with a reception equalizer, and the de-emphasis function by itself cannot sufficiently compensate for the cable loss in the electronic endoscope which has small diameter and transmits data over several meters.

In view of the above, an object of the present invention is to provide an electronic endoscope capable of providing cable loss compensation means which enables easy quality control with low power consumption and low cost.

### Disclosure of Invention

### Means for Solving the Problem

An electronic endoscope according to one aspect of the present invention is an electronic endoscope that transmits an image pickup signal obtained by an image pickup section and converted into digital serial data, as a differential signal, to a processor, and the electronic endoscope includes: a first transmission section for transmitting the differential signal to the processor; and a first filter section arranged at an output stage of the first transmission section, wherein a constant of the first filter section is adjusted according to a loss in a transmission channel including a cable such that a quality of output waveform to the processor is fixed.

### Brief Description of the Drawings

FIG. 1 illustrates a configuration of an endoscope system including an electronic endoscope according to a first embodiment.
FIG. 2A describes frequency characteristics.
FIG. 2B describes frequency characteristics.
FIG. 2C describes frequency characteristics.
FIG. 3 illustrates a configuration of an endoscope system including an electronic endoscope according to a second embodiment.
FIG. 4 illustrates a configuration of an endoscope system including an electronic endoscope according to a third embodiment.

### Best Mode for Carrying Out the Invention

Hereinafter, description will be made on embodiments of the present invention.

### (First Embodiment)

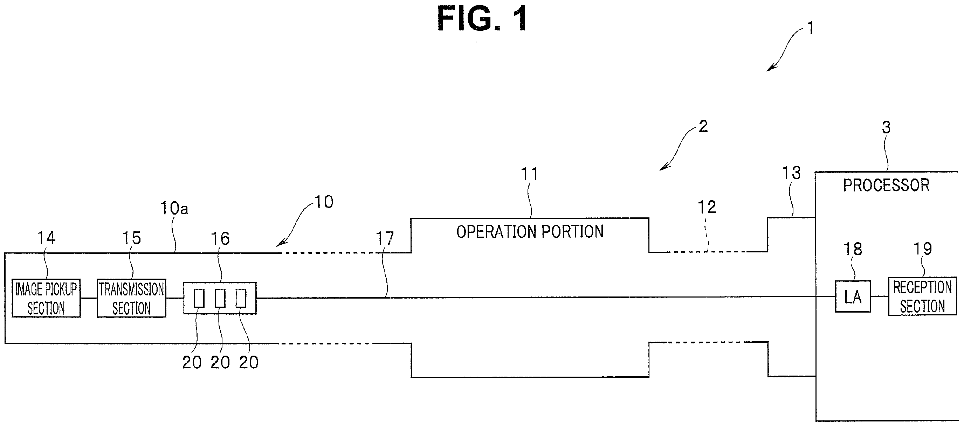

First, with reference to FIG. 1, description will be made on an endoscope system having an electronic endoscope according to the first embodiment. FIG. 1 illustrates a configuration of the endoscope system including the electronic endoscope according to the first embodiment.

As shown in FIG. 1, an endoscope system 1 is configured by including an electronic endoscope 2 according to the present embodiment and a processor 3. The electronic endoscope 2 includes an elongated insertion portion 10 to be inserted into a subject, an operation portion 11 provided on the proximal end side of the insertion portion 10, a universal cable 12 provided on the proximal end side of the operation portion 11, and a connector portion 13 provided on the proximal end side of the universal cable 12. The electronic endoscope 2 is detachably connected to the processor 3 through the connector portion 13.

In addition, the electronic endoscope 2 includes at least an image pickup section 14, a transmission section 15, a filter section 16, and a cable 17. In addition, the processor 3 includes at least a limiting amplifier (hereinafter, referred to as LA) 18, and a reception circuit 19.

At a distal end portion 10a of the insertion portion 10, the image pickup section 14, a transmission section 15, and the filter section 16 are arranged in this order from the distal end side. In addition, the cable 17 is connected to the proximal end side of the filter section 16. The cable 17 is inserted through the insertion portion 10, the operation portion 11, the universal cable 12, and the connector portion 13, and when the electronic endoscope 2 is attached to the processor 3, the cable 17 is connected to the LA 18 in the processor 3.

The image pickup section 14 is configured by an image pickup device such as CCD, and an A/D converter, for example, and generates an image pickup signal by picking up an image of an observation target with the image pickup device, performs A/D conversion on the image pickup signal to convert the image pickup signal from the analog signal to the digital signal with the A/D converter, and outputs the digital signal to the transmission section 15.

The transmission section 15 (first transmission section) converts the image pickup signal, which has been generated and A/D converted by the image pickup section 14, into digital serial data, and thereafter outputs the digital serial data as a differential signal to the processor 3, through the filter section 16.

The filter section 16 is a passive equalizer configured by a passive element which does not require a power source, for example. The filter section 16 is a filter section that is impedance-matched so that the impedance of the filter section is equal to the impedance of the transmission channel including the cable 17. The filter section 16 is configured by at least one or more (three in the example shown in FIG. 1) filters 20 connected in series.

The number of (number of stages of) the filters 20 is adjusted according to the transmission loss in the transmission channel including the cable 17, that is, the transmission loss in the electronic endoscope 2. Therefore, the longer the length of the cable 17, the more the number of the filters 20 in the filter section 16. That is, the constant of the filter section 16 (first filter section) is adjusted according to the loss in the transmission channel including the cable 17 such that the quality of the output waveform to the processor 3 is fixed.

For example, when the length of the cable 17 is short, the number of the filter 20 is one, when the length of the cable 17 is standard, the number of the filters 20 is two, and when the length of the cable 17 is long, the number of the filters 20 is three. The image pickup signal outputted from the filter section 16 is inputted to the LA 18 in the processor 3 through the cable 17.

The LA 18 amplifies the amplitude of the image pickup signal inputted through the cable 17 to predetermined amplitude, and outputs the image pickup signal, the amplitude of which has been amplified, to the reception circuit 19. That is, the LA 18 amplifies the amplitude lowered by the filter section 16 which does not require a power source to required amplitude, and outputs the image pickup signal, the amplitude of which has been amplified to the required amplitude, to the reception circuit 19.

The reception circuit 19 receives the image pickup signal from the LA 18 with an internal clock, and thereafter converts the received image pickup signal to parallel data. The image pickup signal converted into the parallel data is subjected to predetermined image processing in an image processing circuit, not shown, and thereafter outputted to a monitor or recording apparatus, not shown. Then, an image is displayed or recorded.

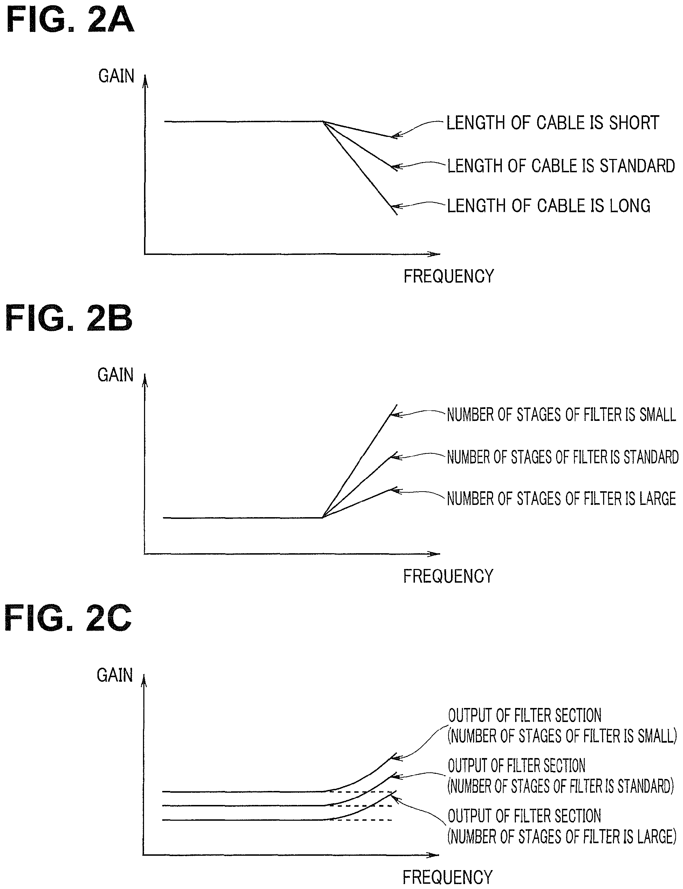

Next, the working of the endoscope system 1 thus configured will be described. FIGS. 2A, 2B, and 2C describe frequency characteristics.

FIG. 2A shows an example of the frequency characteristic of the cable 17, with the horizontal axis representing the frequency and the vertical axis representing the gain. The cable 17 has a characteristic similar to that of a low-pass filter, and allows the components of frequency lower than a certain frequency to pass through, and attenuates the components of the frequency equal to or higher than the certain frequency. Therefore, when the frequency becomes equal to or higher than the certain frequency, the gain becomes smaller. In addition, the longer the length of the cable 17, the more the high-frequency components attenuate. As a result, the gain becomes smaller.

Furthermore, FIG. 2B shows an example of the frequency characteristic of the filter section 16, with the horizontal axis representing the frequency and the vertical axis representing the gain. The filter section 16, which is a passive equalizer, has a characteristic similar to that of a high-pass filter or band-pass filter, and attenuates the components of the frequency lower than the certain frequency and allows the components of the frequency equal to or higher than the certain frequency to pass through. Therefore, when the frequency becomes equal to or higher than the certain frequency, the gain becomes relatively large. In addition, the larger the number of the stages of the filters 20, the more the filter section 16 attenuates the low-frequency components, which results in a large gain difference between the low frequency and the high frequency.

FIG. 2C shows an example of the frequency characteristic of the image pickup signal outputted from the filter section 16, with the horizontal axis representing the frequency and the vertical axis representing the gain. According to the frequency characteristic of the filter section 16 in FIG. 2B, the gain of the image pickup signal outputted from the filter section 16 becomes large when the frequency becomes equal to or larger than the certain frequency as shown in FIG. 2C. The image pickup signal outputted from the filter section 16 is transmitted to the LA 18 through the cable 17, and the gain of the image pickup signal becomes small, when the frequency becomes equal to or larger than the certain frequency, due to the characteristic of the cable 17 similar to that of the low-pass filter as shown in FIG. 2A. That is, as shown by the dashed lines in FIG. 2C, the image pickup signal whose gain is substantially flat is inputted to the processor 3.

As described above, since the filter section 16 is configured by a passive element in the electronic endoscope 2, the filter section does not require a power source, which enables the power consumption to be reduced. In addition, the electronic endoscope 2 is not required to have a power source for the filter section 16, which does not cause heat generation. That is, no additional heat-radiation mechanism is required, which enables the diameter and the size of the electronic endoscope 2 to be reduced.

Furthermore, generally, the signal is transmitted by the cable and thereafter passed through the equalizer. In the present embodiment, however, the cable 17 has the characteristic similar to that of the low-pass filter and the filter section 16 has the characteristic similar to that of the high-pass filter or band-pass filter, and both the cable 17 and the filter section 16 are passive elements and impedances are matched. Therefore, even if the transmission order is reversed, the overall transfer function is the same. Therefore, according to the electronic endoscope 2, the characteristic of the filter section 16 is adjusted in accordance with the transmission loss in the transmission channel including the cable 17, which leads to a decrease in the amplitude at the input end of the processor 3, but enables a stable waveform to be obtained, with the intersymbol interference being suppressed.

In addition, according to the electronic endoscope 2, the low-frequency noise can be reduced due to the characteristic of the filter section 16 before the transmission of the signal by the cable 17, which enables the electromagnetic radiation to be suppressed.

Therefore, the electronic endoscope according to the present embodiment is capable of providing cable loss compensation means that facilitates quality control with low power consumption and low cost.

### (Second Embodiment)

Next, description will be made on the second embodiment.

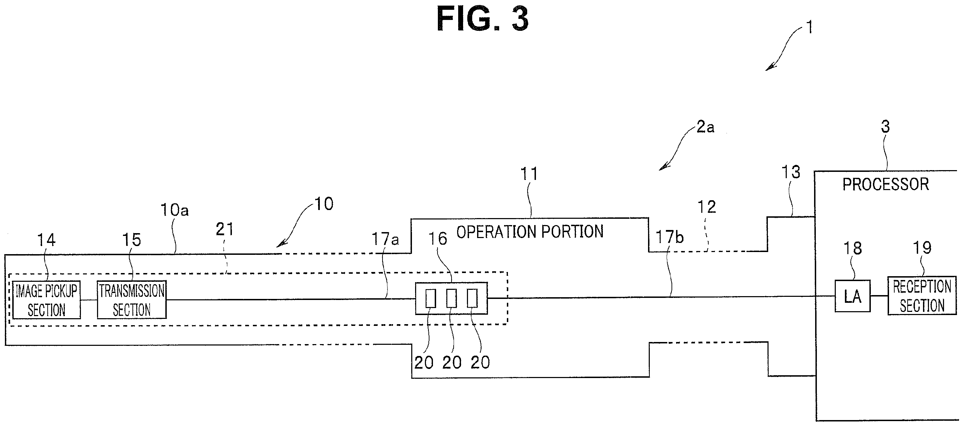

FIG. 3 illustrates a configuration of an endoscope system including an electronic endoscope according to a second embodiment. Note that the constituent elements in FIG. 3 which are same as those in FIG. 1 are attached with the same reference numerals, and description thereof will be omitted.

As shown in FIG. 3, an electronic endoscope 2a according to the present embodiment includes the filter section 16 arranged in the operation portion 11. The filter section 16 is connected to the transmission section 15 through a cable 17a, and connected to the LA 18 through a cable 17b. Note that the filter section 16 is connected to the transmission section 15, through the cable 17a. However, the filter section 16 may be connected to the transmission section 15 through an impedance-controlled electric wire or flexible substrate, for example.

Thus, also in the electronic endoscope 2a according to the present embodiment, the number (number of stages) of the filters 20 of the filter section 16 is adjusted according to the loss in the transmission channel including the cables 17a, 17b. According to such a configuration, the same effects as those in the first embodiment can be obtained.

In addition, in the electronic endoscope 2a according to the present embodiment, the filter section 16 is arranged in the operation portion 11 which has a larger substrate area compared with the insertion portion 10, which enables the filters 20 to be configured by a plurality of circuits.

Furthermore, according to the electronic endoscope 2a of the present embodiment, the filter section 16 has only to be adjusted, with the part surrounded by the dashed lines as one unit 21, only when the length of the cable 17a is changed according to the types of the electronic endoscope 2, for example. Therefore, quality control at the time of assembly and repair is easy.

### (Third Embodiment)

Next, description will be made on the third embodiment.

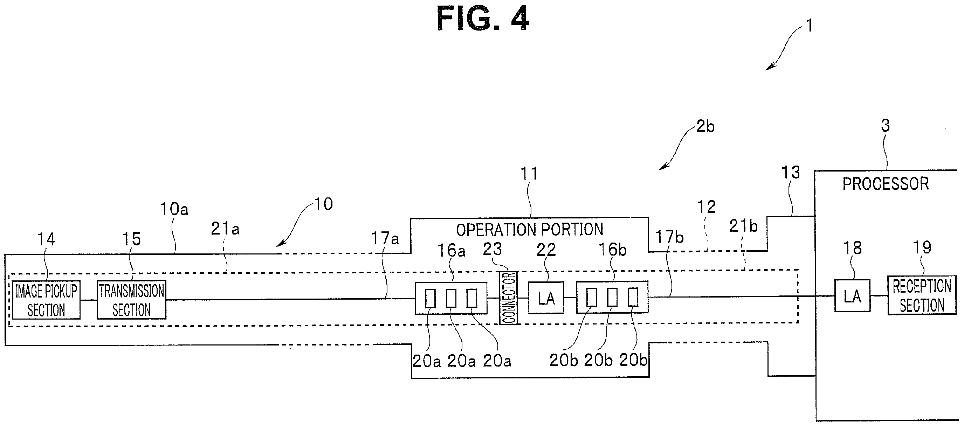

FIG. 4 illustrates a configuration of an endoscope system including an electronic endoscope according to the third embodiment. Note that the constituent elements in FIG. 4 which are same as those in FIG. 3 are attached with the same reference numerals, and description thereof will be omitted.

As shown in FIG. 4, an electronic endoscope 2b according to the present embodiment includes a filter section 16a, an LA 22, and a filter section 16b in the operation portion 11. The filter section 16a is connected to the transmission section 15 through the cable 17a, and the filter section 16b is connected to the LA 18 through the cable 17b.

The filter section 16a is configured by a plurality of filters 20a, and the number (number of stages) of the filters 20a are adjusted according to the loss in the transmission channel including the cable 17a. The LA 22 as a second transmission section amplifies the amplitude of the image pickup signal inputted through the filter section 16a to predetermined amplitude, and outputs the image pickup signal, the amplitude of which has been amplified, to the filter section 16b. Then, the filter section 16b (second filter section) is configured by a plurality of filters 20b, and the number (the number of stages) of the filters 20b is adjusted according to the loss in the transmission channel including the cable 17b.

Thus, also in the electronic endoscope 2b according to the present embodiment, the number (the number of stages) of the filters 20a of the filter section 16a is adjusted according to the loss in the transmission channel including the cable 17a, and the number (the number of stages) of the filters 20b of the filter section 16b is adjusted according to the loss in the transmission channel including the cable 17b. According to such a configuration, the same effects as those in the first embodiment can be obtained.

In addition, in the electronic endoscope 2b according to the present embodiment, the filter sections 16a, 16b and the LA 22 are arranged intensively in the operation portion 11 which has a larger substrate area compared with the insertion portion 10, which facilitates the size reduction of the insertion portion 10.

Furthermore, in the electronic endoscope 2b according to the present embodiment, a unit 21a and a unit 21b which are the parts surrounded by the dashed lines may be connected to each other through the connector 23. Then, if the filter section 16a is adjusted in accordance with the loss in the transmission channel including the cable 17a in the unit 21a and the filter section 16b is adjusted in accordance with the loss in the transmission channel including the cable 17b in the unit 21b, the quality control can be easily performed at the time of assembly and repair.

The present invention is not limited to the above-described embodiments and various changes, modifications, and the like are possible in a range without changing the gist of the present invention.

The present application claims the benefit of Japanese Patent Application No. 2014-97061 filed in Japan on May 8, 2014, the entire disclosure contents of which are incorporated in the description, claims, and drawings of the present application by reference.

## Claims

1. An electronic endoscope that transmits an image pickup signal obtained by an image pickup section and converted into digital serial data, as a differential signal, to a processor, the electronic endoscope comprising:
a first transmission section for transmitting the differential signal to the processor; and
a first filter section arranged at an output stage of the first transmission section, wherein a constant of the first filter section is adjusted according to a loss in a transmission channel including a cable such that a quality of output waveform to the processor is fixed.

2. The electronic endoscope according to claim 1, wherein the first filter section is impedance-controlled such that an impedance of the first filter section is equal to an impedance of the transmission channel.

3. The electronic endoscope according to claim 1, wherein the first filter section is configured by at least one or more filters connected in series, and the constant is adjusted according to a number of stages of the one or more filters.

4. The electronic endoscope according to claim 1, wherein the first transmission section and the first filter section are arranged in an insertion portion.

5. The electronic endoscope according to claim 1, wherein the first transmission section is arranged in an insertion portion, and the first filter section is arranged in an operation portion or in a portion subsequent to the operation portion through an electric wire or a flexible substrate.

6. The electronic endoscope according to claim 1, further comprising:
a second transmission section for transmitting the differential signal from the first filter section to the processor; and
a second filter section arranged at an output stage of the second transmission section, wherein a constant of the second filter section is adjusted according to a loss including a cable located in the second transmission section or in a portion subsequent to the second transmission section,
wherein the first filter section, the second transmission section, and the second filter section are arranged in an operation portion or in a portion subsequent to the operation portion.

7. The electronic endoscope according to claim 6, wherein the first filter section, the second transmission section, and the second filter section are arranged intensively in the operation portion.
